# EUROPEAN PATENT APPLICATION

(11) **EP 4 184 166 A1**
(43) Date of publication of application: **24.05.2023**
(21) Application number: 21845811.5
(22) Date of filing: 20.07.2021
(51) Int. Cl.: G01N 33/50, G01N 33/68, C12Q 1/6886, A61K 38/17, A61P 35/04

(54) **METHOD FOR SCREENING COLORECTAL CANCER METASTASIS INHIBITOR**

(30) Priority: 20.07.2020 KR 20200089603
(71) Applicant: FNCT Biotech, Inc., Seoul 04626 (KR)
(72) Inventor: LEE, Su Jae, Seoul 03168 (KR); LIM, Eun Ji, Seoul 04761 (KR)
(74) Representative: Dr. Schön, Neymeyr & Partner Patentanwälte mbB
(86) International application number: PCT/KR2021/009367
(87) International publication number: WO 2022/019626

(57) **Abstract**

The present disclosure relates to a method for screening a colorectal cancer inhibitor and, more specifically, to a method for screening a colorectal cancer inhibitor wherein a material increasing a binding level of ICAM-1 to SRC is selected as a therapeutic material. In the present disclosure, ICAM-1 is identified to regulate cancer metastasis and angiogenesis in an SRC-dependent manner, thereby exhibiting that ICAM-1 can be a potential target factor for developing a colorectal cancer therapeutic agent. Accordingly, a colorectal cancer therapeutic agent employing the target factor screened by the method of the present disclosure can increase the therapeutic effect for colorectal cancer patients.

## Description

### Technical Field

The present disclosure relates to a method for screening colorectal cancer metastasis inhibitors. More particularly, the present disclosure relates to a method for screening colorectal cancer metastasis inhibitors that select materials that increase the binding level of ICAM-1 to SRC as therapeutic materials.

### Background Art

Colorectal cancer is the third most diagnosed cancer in the world and is known to be the fourth most common cause of death, and the mortality rate of colorectal cancer in Korea is increasing gradually, reaching 73%. Colorectal cancer may be classified into sporadic colorectal cancer and colitis-associated colorectal cancer, depending on the incidence factor. Sporadic colorectal cancer is caused by several factors such as genetic factors, aging, type 2 diabetes, overweight, drinking, and smoking, and colitis-associated colorectal cancer is mainly caused by continuous inflammatory bowel diseases such as Crohn's disease and ulcerative colitis. Since sporadic colorectal cancer is mostly caused by aging or genetic factors, it mainly appears in elderly patients, but colitis-associated colorectal cancer is more likely to occur in younger patients and is known to have a higher mortality rate than sporadic colorectal cancer.

On the other hand, colonoscopy is a common screening method to identify and remove polyps, but colonoscopy causes many side effects such as intestinal perforation, intestinal bleeding, and sleep apnea, and there are many difficulties in accurately screening tumors. Therefore, there is a need to develop a marker for diagnosing or treating colorectal cancer.

Recently, there is a study report that inhibition of E3 ligase regulation increases growth, metastasis, and cancer stem cell proliferation in colorectal cancer (Christina L et al., Surgery, 2007, June, 705-707. (2007.06)), but specifically, the mechanism for metastasis and angiogenesis of cancer cells is not yet known.

### Disclosure

### Technical Problem

In order to solve the above problems, the present inventors completed the present disclosure by confirming that ICAM-1 controls cancer metastasis and angiogenesis dependent on SRC as a result of studying candidate materials for colorectal cancer metastasis inhibitors.

Accordingly, an objective of the present disclosure is to provide a method for screening a colorectal cancer metastasis inhibitor.

In addition, another objective of the present disclosure is to provide a composition for predicting colorectal cancer metastasis, which includes an agent for measuring a mRNA level of the ICAM-1 gene or an agent for measuring a level of a protein that the gene encodes.

The other objective of the present disclosure is to provide a pharmaceutical composition for inhibiting colorectal cancer metastasis, which contains the ICAM-1 gene or the protein that the gene encodes, as an active ingredient.

However, the technical problem to be achieved by the present disclosure is not limited to the above-mentioned problems, and other problems not mentioned will be clearly understood by those skilled in the art from the following description.

### Technical Solution

In order to achieve the above objectives, the present disclosure provides a method for screening colorectal cancer metastasis inhibitors, the method comprising: (a) treating cells with a candidate material in vitro; (b) measuring the binding level of intracellular adhesion molecule 1 (ICAM-1) to SRC in the cell; and (c) selecting a material that increases the binding level of ICAM-1 and SRC as a therapeutic material, compared to the non-treatment group of the candidate material.

In one embodiment of the present disclosure, the cell may be a colorectal cancer cell.

In another embodiment of the present disclosure, the candidate material may be selected from the group consisting of compounds, microbial cultures or extracts, natural product extracts, nucleic acids, and peptides.

In still another embodiment of the present disclosure, the nucleic acid may be selected from the group consisting of siRNA, shRNA, microRNA, antisense RNA, aptamer, locked nucleic acid (LNA), peptide nucleic acid (PNA), and morpholino.

In still another embodiment of the present disclosure, the measurement of the binding level of ICAM-1 to SRC may be measuring the mRNA level of ICAM-1 and SRC genes.

In still another embodiment of the present disclosure, the mRNA level may be measured by at least one method selected from the group consisting of polymerase chain reaction (PCR), reverse transcription polymerase chain reaction (RT-PCR), real-time polymerase chain reaction (real-time PCR), RNase protection assay (RPA), microarray, and northern blotting.

In addition, the present disclosure provides a composition for predicting colorectal cancer metastasis, which includes an agent for measuring the mRNA level of the ICAM-1 gene or an agent for measuring the level of the protein that the gene encodes.

In addition, the present disclosure provides a pharmaceutical composition for inhibiting colorectal cancer metastasis, which contains the ICAM-1 gene or the protein that the gene encodes as an active ingredient.

### Advantageous Effects

The present disclosure showed that ICAM-1 can be a potential target for the development of colorectal cancer therapeutics by confirming that ICAM-1 can regulate cancer metastasis and angiogenesis in an SRC-dependent manner. Accordingly, it is possible to increase the therapeutic effect of colorectal cancer patients by using the colorectal cancer therapeutic agent using a target factor screened by the method of the present disclosure.

### Description of Drawings

FIG. 1A shows a result of confirming the difference in ICAM-1 expression between normal people and inflammatory bowel disease patients.
FIG. 1B shows a result of confirming the difference in ICAM-1 expression between normal people and cancer patients.
FIG. 1C shows a result confirming that the expression of ICAM-1 is increased in colorectal cancer patients through immunohistochemistry(IHC) experiments.
FIG. 1D shows a result of confirming the expression level of ICAM-1 in colorectal cancer cells by performing real-time PCR on a normal cell line CCD841 and colorectal cancer cell lines (HT-29, HCT-116, SW-620, SW-480, DLD-1).
FIGS. 1E and 1F show the results of confirming the relationship between colorectal cancer malignancy and ICAM-1 through the CPTAC database and colorectal cancer patient tissue data.
FIGS. 1G and 1H show the results of analyzing the data used in FIGS. 1E and 1F, which are confirming that the higher the expression of ICAM-1, the lower the survival rate and the disease-specific survival rate.
FIGS. 1I and 1J show the results of confirming the expression level of ICAM-1 in a patient in which metastasis to lymph nodes has occurred.
FIG. 2A shows a result of confirming the relationship between the high expression level of ICAM-1 and tumor metastasis and angiogenesis using GSEA analysis.
FIG. 2B is a schematic diagram showing a method of injecting the SW-480 cell line with reduced ICAM-1 expression into the cecum of mice.
FIG. 2C shows a result of confirming the relationship between ICAM-1 expression and tumor metastasis in the mouse transplanted with the SW-480 cell line in FIG. 2B.
FIGS. 2D and 2E show the results of confirming the expression levels of EMT markers and metastasis factors etc. in the mouse transplanted with the SW-480 cell line in FIG. 2B.
FIG. 2F shows a result of confirming the relationship between the expression level of ICAM-1 and angiogenesis in vitro.
FIGS. 2G and 2H show the results of confirming the relationship between the expression level of ICAM-1 and angiogenesis in the mouse model of FIG. 2B in vivo through PCR and tissue immunofluorescence.
FIG. 3A shows a result of confirming a correlation between the ICAM-1 and the p-SRC by performing a kinase array on the ICAM-1.
FIGS. 3B and 3C show the results of confirming the SRC activity according to ICAM-1 expression through the TCGA database for colorectal cancer patients.
FIG. 3D shows a result of confirming the interaction between ICAM-1 and SRC using the immunoprecipitation method.
FIG. 3E shows a result of confirming the binding position of SRC to ICAM-1 by performing tissue immunofluorescence in order to specifically confirm the interaction between ICAM-1 and SRC.
FIGS. 3F and 3G show the results of confirming the relationship between tumor metastasis and angiogenesis after producing a mutation that inhibits or activates the Tyr512 residue of ICAM-1.
FIG. 4A shows a result of confirming the gene exhibiting high expression in common in colorectal cancer patients through four GEO databases.
FIG. 4B shows a result of specifically confirming the expression of ICAM-1 and SRC after treatment with a c-MET inhibitor.
FIG. 4C shows a result of confirming an expression level of p-SRC when HGF, which is a ligand of c-MET, is treated after inhibiting the expression of ICAM-1.
FIG. 4D shows a result of confirming the activation of SRC in the mutant overexpressing ICAM-1.
FIGS. 4E and 4F show the results of observing the binding level of c-MET to SRC according to the reduction of ICAM-1, through IP and PLA.
FIG. 4G shows a result of confirming the expression level of p-SRC through ICC when ICAM-1 and c-MET are present at the same position.
FIG. 5A shows a result of confirming the metastasis when the SRC is knocked down after overexpressed ICAM-1.
FIGS. 5B and 5C show the results of confirming the expression levels of EMT markers and transcription factors through real-time PCR and western blot when the SRC is knocked down after overexpressed ICAM-1.
FIGS. 5D, 5E, and 5F show the results of confirming the angiogenesis and the expression of growth factors that induce the angiogenesis when the SRC is knocked down after overexpressing ICAM-1.
FIG. 5G shows a result of confirming the correlation between the expression level of ICAM-1 and p-SRC in the tissue of a colorectal cancer patient.
FIG. 5H shows a result of confirming the correlation between the expression levels of ICAM-1 and p-SRC and the prognosis of colorectal cancer patients.
FIG. 6A shows a result of confirming the metastatic ability of the tumor when 3 weeks have elapsed after injecting the tumor into the cecum and then injecting the monoclonal antibody into the abdominal cavity.
FIG. 6B and 6C show the results of confirming the expression level of metastasis-related genes by PCR and tissue immunostaining method when 3 weeks have elapsed after injecting the tumor into the cecum.
FIG. 6D shows a result of confirming the angiogenesis ability of blood vessels when a monoclonal antibody is treated on an SW-480 cell line.
FIGS. 6E and 6F show the results of the expression level and angiogenesis level of growth factors that control angiogenesis in vivo when treated with monoclonal antibodies.

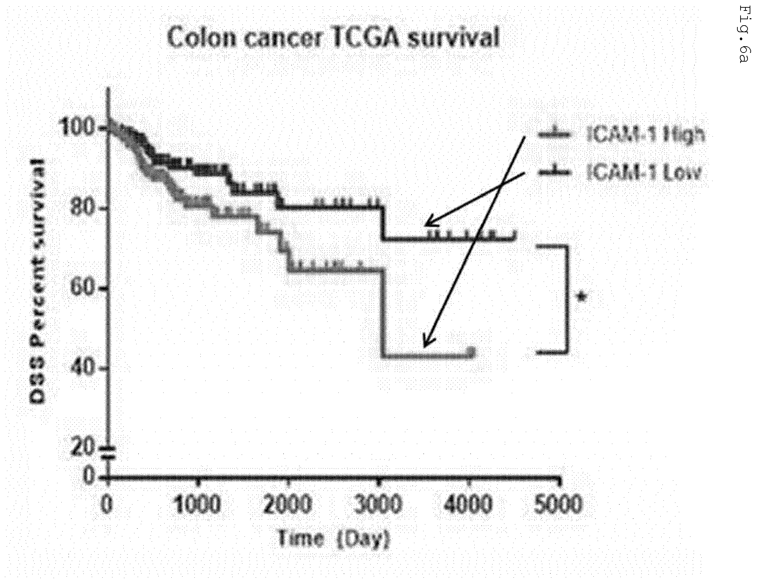

FIG. 6A shows a result of confirming the survival rate of colorectal cancer according to ICAM-1 expression.

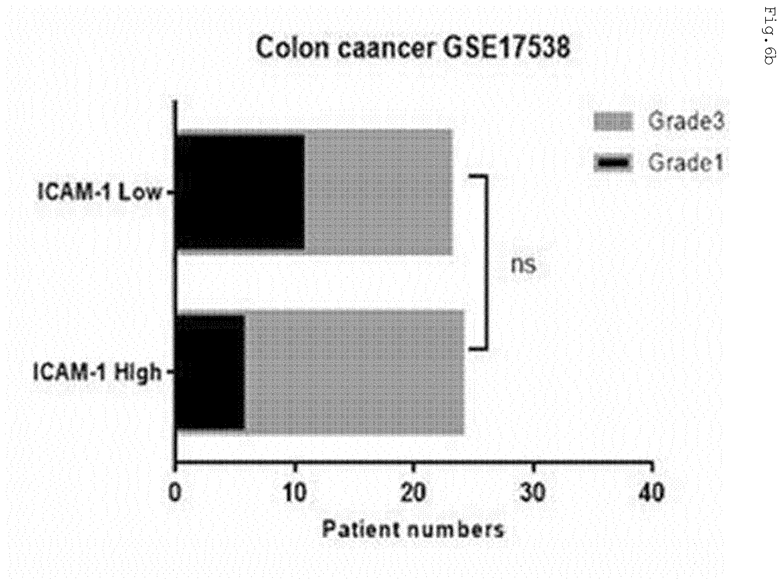

FIG. 6B shows a result of analyzing the colorectal cancer grade according to ICAM-1 expression.

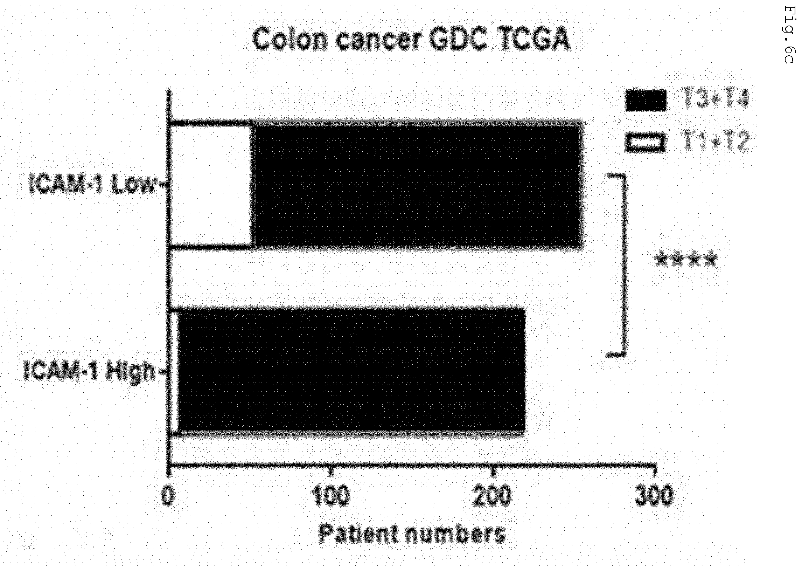

FIG. 6C shows a result of analyzing the tumor size according to ICAM-1 expression.

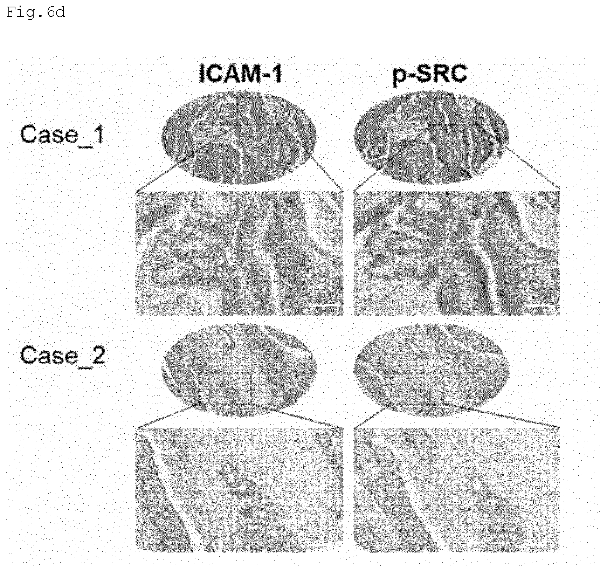

FIG. 6D shows a result of confirming the correlation between ICAM-1 and p-SRC in the tissue of a colorectal cancer patient.

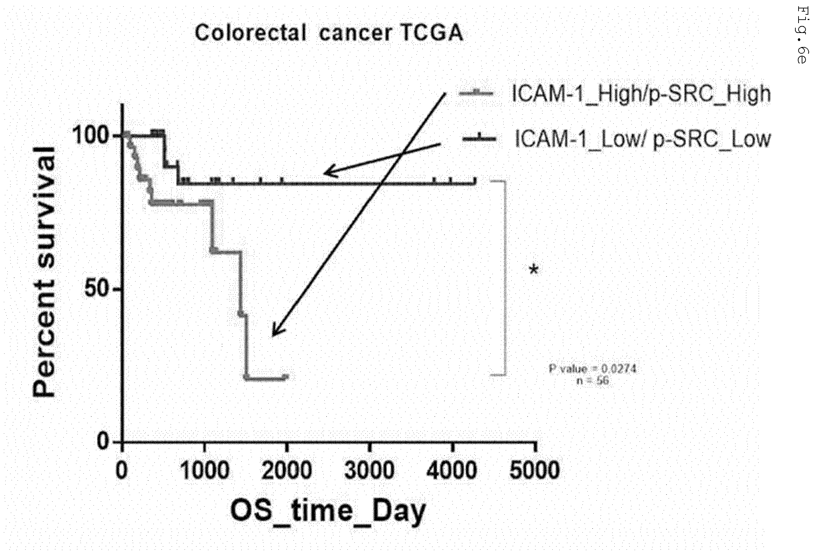

FIG. 6E shows a result of confirming a prognosis according to an increase in expression of ICAM-1 and p-SRC.

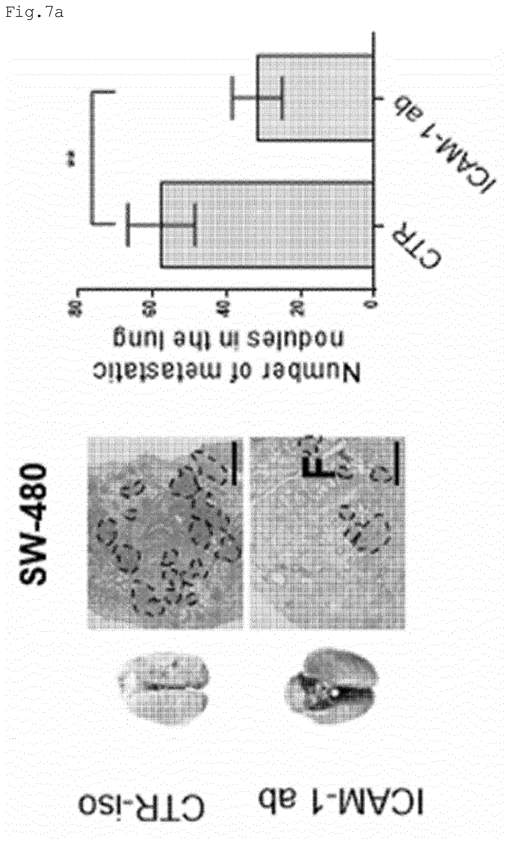

FIG. 7A shows a result of confirming the metastasis of the tumor after injecting the tumor into the cecum and then injecting the monoclonal antibody into the abdominal cavity.

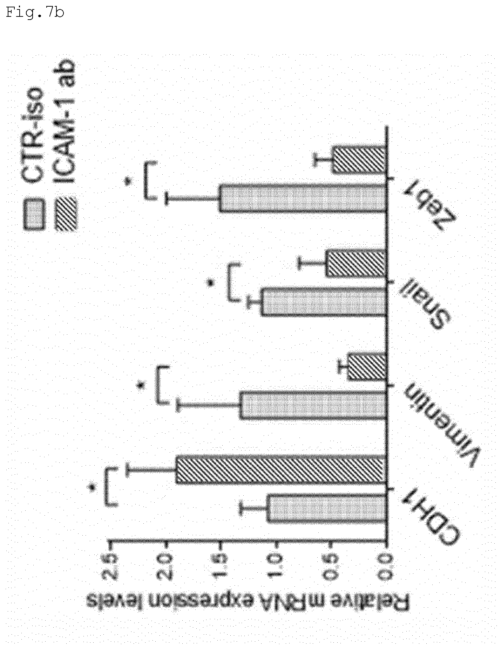

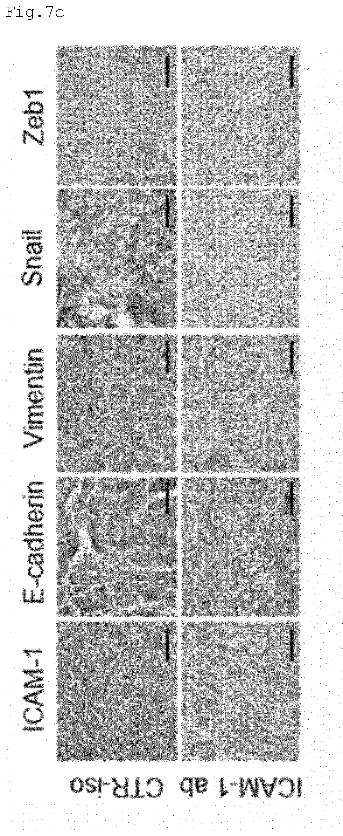

FIGS. 7B and 7C show the results of confirming the expression level of metastasis-related genes through PCR and tissue immunostaining after injecting the tumor into the cecum.

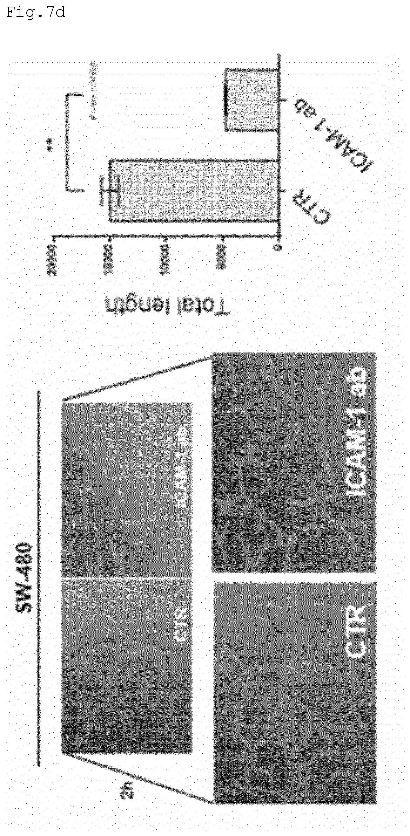

FIG. 7D shows a result of confirming the angiogenesis of blood vessels after treating ICAM-1 monoclonal antibody on an SW-480 cell line.

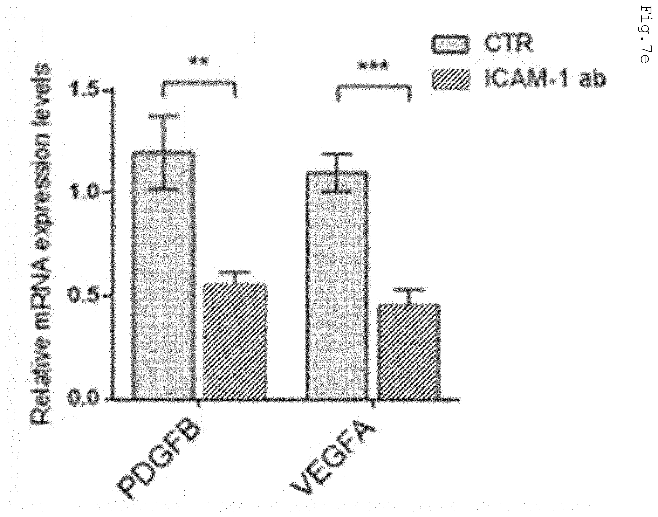

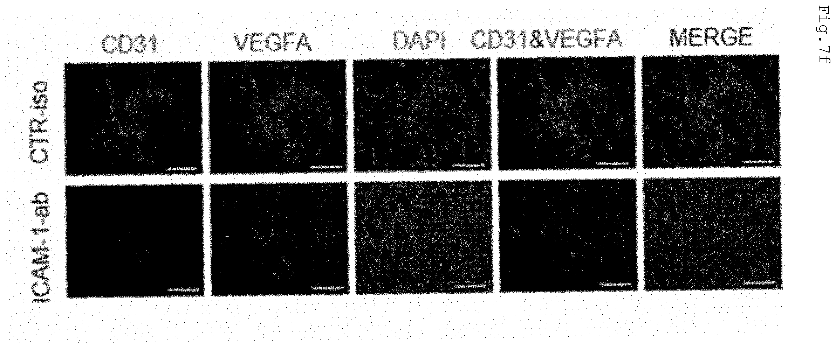

FIGS. 7E and 7F show the results of confirming the expression of a factor controlling angiogenesis in vivo and the number of newly created blood vessels.

### Best Mode

As a result of studying candidate material for colorectal cancer metastasis inhibitors, the present inventors completed the present disclosure by confirming that ICAM-1 regulates cancer metastasis and angiogenesis dependent on SRC.

Accordingly, the present disclosure provides a method for screening colorectal cancer metastasis inhibitors, the method comprising: (a) treating cells with a candidate material in vitro; (b) measuring the binding level of intracellular adhesion molecule 1 (ICAM-1) to SRC in the cell; and (c) as a therapeutic material, selecting a material that increases the binding level of ICAM-1 and SRC compared to the non-treatment group of the candidate material.[64] In the present disclosure, "inhibition of colorectal cancer metastasis" means not only inhibiting the cell proliferation caused by colorectal cancer but also inhibiting metastasis and mobility of colorectal cancer. The colorectal cancer metastasis inhibitor may include any molecule, such as, for example, proteins, oligopeptides, small organic molecules, polysaccharides, polynucleotides, and a wide range of compounds.

In the present disclosure, the "cell" may be a colon tissue-derived cell, but is not limited thereto, and is used to compare the binding level of ICAM-1 to SRC, and may preferably be a colorectal cancer cell, but is not limited thereto.

The term "candidate material" used in the present disclosure means a material expected to increase the binding level of ICAM-1 to SRC, and the candidate material may be selected from the group consisting of compounds, microbial cultures or extracts, natural product extracts, nucleic acid, and peptides, but is not limited thereto.

In the present disclosure, the nucleic acid is selected from the group consisting of siRNA, shRNA, microRNA, antisense RNA, aptamer, locked nucleic acid (LNA), peptide nucleic acid (PNA), and morpholino, but is not limited thereto.

The measurement of the binding level of ICAM-1 to SRC according to the present disclosure may be measuring the mRNA level of ICAM-1 and SRC genes.

In the present disclosure, the mRNA level may be measured by at least one method selected from the group consisting of polymerase chain reaction (PCR), reverse transcription polymerase chain reaction (RT-PCR), real-time polymerase chain reaction (real-time PCR), RNase protection assay (RPA), microarray, and northern blotting.

The present inventors have identified that ICAM-1 controls metastatic and angiogenesis dependent on SRC through specific embodiments.

In one embodiment of the present disclosure, it was confirmed that SRC was decreased when expression of ICAM-1 was inhibited, and that the activity of SRC was increased when ICAM-1 was expressed, and it was also confirmed that the Tyr512 residue of ICAM-1 is bound to SRC, through an immunoprecipitation reaction.(see Example 3).

In another embodiment of the present disclosure, when SRC was knocked down after overexpression of ICAM-1, it was confirmed that even though the expression of ICAM-1 was increased, the metastasis decreased as the SRC decreased. By confirming that the expression of EMT markers and transcription factors, and the expression level of angiogenesis-inducing growth factor decreased, it was confirmed that ICAM-1 regulates metastasis and angiogenesis dependent on SRC (see Example 4).

From the above Example, it may be confirmed that ICAM-1 is involved in the metastasis and angiogenesis of colorectal cancer, and it may be inferred that the colorectal cancer inhibitory efficacy of ICAM-1 is controlled dependent on SRC.

Accordingly, as another aspect of the present disclosure, the present disclosure provides a composition for predicting colorectal cancer metastasis, comprising an agent for measuring the mRNA level of the ICAM-1 gene or an agent for measuring the level of the protein that the gene encodes and a kit comprising the same composition.

In the present disclosure, the ICAM-1 gene may comprise the nucleotide sequence of SEQ ID NO: 2, and a homologue of the nucleotide sequence are also included within the scope of the present disclosure. Specifically, the gene may include a nucleotide sequence having a homology of the nucleotide sequence of 70% or more, preferably 80% or more, more preferably 90% or more, and most preferably 95% or more with the nucleotide sequence of SEQ ID NO: 2,

In addition, the protein which the ICAM-1 gene encodes may comprise the amino acid sequence of SEQ ID NO: 1, and is interpreted to comprise a sequence having a substantial identity to SEQ ID NO: 1. The above substantial identity refers to a sequence representing at least 80%homology, more preferably 90%, 910, 92%, 93%, 94%, and 95% homology, and most preferably 96%, 97%, 98%, and 99% homology when the sequence of the present disclosure and any other sequences are aligned as much as possible, and the aligned sequence is analyzed by using an algorithm commonly used in the art.

In the present disclosure, the agent for measuring the mRNA level may be a sense and anti-sense primer or probe that complementarily binds to the mRNA of the gene.

The term "primer", as a short gene sequence that is the starting point of DNA synthesis, used in this disclosure refers to an oligonucleotide synthesized for use in the diagnosis, DNA sequencing, etc.. The primers may be synthesized and used in a length of 15 to 30 base pairs but may vary according to the purpose of use and may be modified by methylation, capping, or the like, by a known method.

As used herein, the term "probe" refers to several to hundreds of nucleic acids that may specifically bind to mRNAs , which are prepared through an enzymatic chemical separation and purification, or synthesis process. The presence or absence of mRNA can be checked by labeling the probe with a radioactive isotope or enzyme, which can be designed and modified by a known method.

In the present disclosure, the agent for measuring the level of the protein may be an antibody that specifically binds to a protein encoded by a gene, but is not limited thereto.

As used herein, the term "antibody" comprises immunoglobulin molecules having immunological reactivity with a specific antigen and comprises both monoclonal antibodies and polyclonal antibodies. The antibody comprises forms produced by genetic engineering, such as chimeric antibodies (e.g., humanized murine antibodies) and heterogeneous binding antibodies (e.g., bispecific antibodies).

The kit for prediction of the present disclosure is comprised of one or more other component compositions, solutions, or devices suitable for the analysis method.

For example, in order to perform PCR, the kit of the present disclosure may be a kit comprising genomic DNA derived from a sample to be analyzed, a primer set specific to the marker gene of the present disclosure, an appropriate amount of DNA polymerase, a dNTP mixture, a PCR buffer solution, and water. The PCR buffer solution may contain KCl, Tris-HCl, and MgCl₂. In addition, components necessary for performing electrophoresis that can confirm the presence or absence of amplification of the PCR product may be additionally included in the kit of the present disclosure.

In addition, the kit of the present disclosure may be a kit comprising essential elements necessary for performing RT-PCR. In addition to each specific primer pair for the marker gene, the RT-PCR kit may comprise test tubes or other suitable containers, reaction buffers, deoxynucleotides (dNTPs), enzymes such as Taq-polymerases, and reverse transcription enzymes, DNase, RNase inhibitors, DEPC-water, and sterile water. In addition, a primer pair specific for a gene used as a quantitative control group may be included in the RT-PCR kit.

In addition, the kit of the present disclosure may be a kit including essential elements necessary for performing a DNA chip. The DNA chip kit includes a substrate to which a gene or a cDNA corresponding to a fragment thereof is attached as a probe, and the substrate may comprise a quantitative structure gene or a cDNA corresponding to a fragment thereof. In addition, the kit of the present disclosure may be in the form of a microarray having a material on which the marker gene of the present disclosure is immobilized.

In addition, the kit of the present disclosure may be a kit comprising essential elements necessary for performing ELISA. The ELISA kit comprises an antibody specific for a marker protein and an agent for measuring the level of the protein. The ELISA kit may comprise a reagent capable of detecting an antibody that forms an "antigen-antibody complex", for example, a labeled secondary antibody, chromophores, an enzyme, and a substrate thereof. It may also comprise antibodies specific to proteins as quantitative control.

As used herein, the term "antigen-antibody complex" refers to a combination of a protein encoded by a gene and an antibody specific thereto. The amount of formation of the antigen-antibody complex can be quantitatively measured through the magnitude of the signal of the detection label. The detection label may be selected from the group consisting of an enzyme, a fluorescent material, a ligand, a luminescent material, a microparticle, a redox molecule, and a radioisotope, but is not limited thereto.

As another aspect of the disclosure, the disclosure provides information provision method for predicting colorectal cancer metastasis, comprising measuring the expression level of the mRNA of the ICAM-1 gene or the protein encoded by the gene in a biological sample derived from a subject.

The term "a method of providing information for predicting colorectal cancer metastasis" used in the present disclosure provides objective basic information necessary for the diagnosis of colorectal cancer metastasis as a preliminary step for diagnosis, and a clinical judgment or opinion of a doctor is excluded.

The biological sample derived from the subject may comprise tissues, cells, whole blood, blood, saliva, sputum, cerebrospinal fluid, and urine but is not limited thereto.

The expression level of the mRNA may be measured by at least one method selected from the group consisting of polymerase chain reaction (PCR), reverse transcription polymerase chain reaction (RT-PCR), real-time polymerase chain reaction (real-time PCR), RNase protection assay (RPA), microarray, and northern blotting, but is not limited thereto.

The protein expression level may be measured according to a conventional method known in the art by at least one method selected from the group consisting of western blotting, radioimmunoassay (RIA), radioimmunodiffusion, enzyme immunoassay (ELISA), immunoprecipitation, flow cytometry, immunofluorescence, ouchterlony, complement fixation assay, and protein chip, but is not limited thereto.

As another aspect of the present disclosure, the present disclosure provides a pharmaceutical composition for inhibiting colorectal cancer metastasis, comprising the ICAM-1 gene or a protein encoded by the gene as an active ingredient.

The term "metastasis inhibition" used in this disclosure refers to all actions that delay the development of colorectal cancer, comprising inhibition of metastasis and angiogenesis of colorectal cancer by administration of the pharmaceutical composition according to this disclosure.

The pharmaceutical composition, according to the present disclosure, comprises an ICAM-1 gene or a protein encoded by the gene as an active ingredient and may further comprise a pharmaceutically acceptable carrier. The pharmaceutically acceptable carrier is commonly used in the formulation and comprises saline, sterile water, Ringer's solution, buffered saline, cyclodextrin, dextrose solution, maltodextrin solution, glycerol, ethanol, liposome, and the like, but is not limited thereto and may further comprise other conventional additives such as antioxidants and buffers, if necessary. In addition, diluents, dispersants, surfactants, binders, lubricants, etc., may be additionally added to form an injectable formulation such as an aqueous solution, suspension, emulsion, etc., pills, capsules, granules, or tablets.

The pharmaceutical composition of the present disclosure may be administered orally or parenterally (for example, intravenously, subcutaneously, intraperitoneally, or locally applied) according to the desired method, but is preferably administered orally. The dosage varies depending on the condition and weight of the patient, the severity of the disease, the drug form, the administration route, and time, but it may be appropriately selected by those skilled in the art.

The pharmaceutical composition of the present disclosure is administered in a pharmaceutically effective amount. In the present disclosure, "pharmaceutically effective amount" means an amount sufficient to treat or diagnose a disease at a reasonable benefit/risk ratio applicable to medical treatment or diagnosis. The effective dose level will depend on the patient's disease type, severity, drug activity, sensitivity to a drug, administration time, administration route and excretion rate, duration of treatment, factors comprising concomitant drugs, and other factors well known in the medical field. The pharmaceutical composition, according to the present disclosure, may be administered as an individual therapeutic agent or in combination with other therapeutic agents, may be administered sequentially or simultaneously with conventional therapeutic agents, and may be administered single or multiple. It is important to administer an amount that can obtain the maximum effect in a minimum amount without side effects in consideration of all the above factors, which may be easily determined by those skilled in the art.

Specifically, the effective amount of the pharmaceutical composition of this disclosure varies depending on the patient's age, gender, state, weight, absorption of active ingredients in the body, inertness rate and excretion rate, disease type, and concomitant drugs. In general, 0.001 to 150 mg per 1 kg of weight, preferably 0.01 to 100 mg, can be administered daily or every other day, or divided into 1 to 3 times a day. However, since it can be increased or decreased depending on the route of administration, the severity of obesity, gender, weight, and age etc., the above dose is not limited to the scope of this disclosure in any way.

Meanwhile, the present disclosure provides a method for predicting colorectal cancer metastasis using the composition for predicting colorectal cancer metastasis.

The present disclosure also provides a method for preventing, inhibiting, or treating colorectal cancer metastasis, comprising a method of administering the pharmaceutical composition to an individual.

In addition, the present disclosure provides use for predicting colorectal cancer metastasis, of the composition for predicting colorectal cancer metastasis.

In addition, the present disclosure provides a use for preventing, inhibiting, or treating colorectal cancer metastasis of the pharmaceutical composition.

Hereinafter, the present disclosure will be described in detail through examples. However, the Examples of the present disclosure are intended to illustrate the present disclosure, and the scope of the present disclosure is not limited to these Examples.

### [Example]

### Example 1. Confirmation of ICAM-1 expression in colorectal disease and colorectal cancer

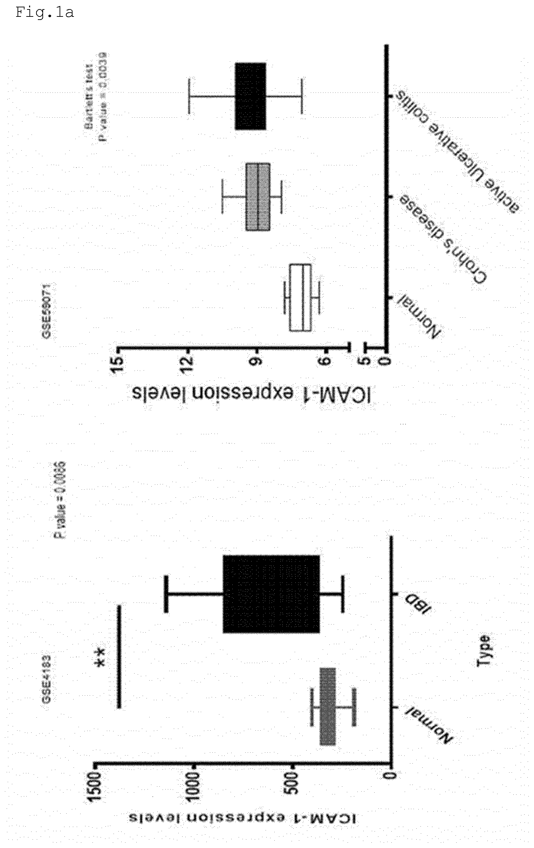

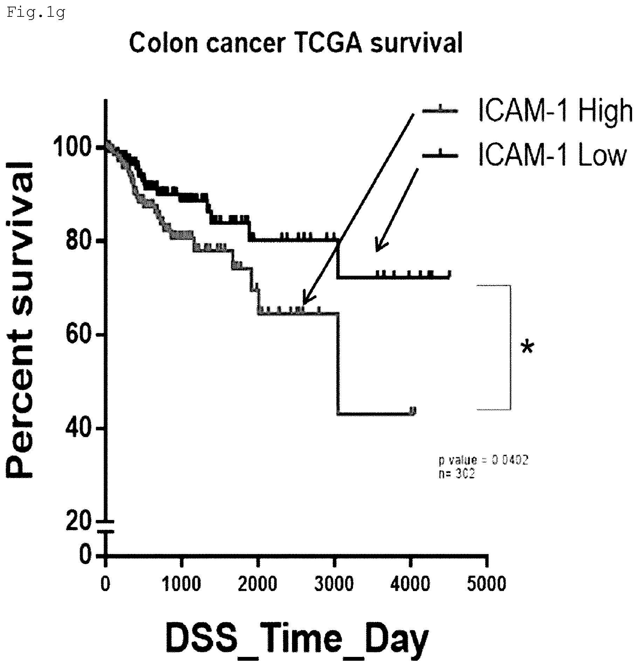

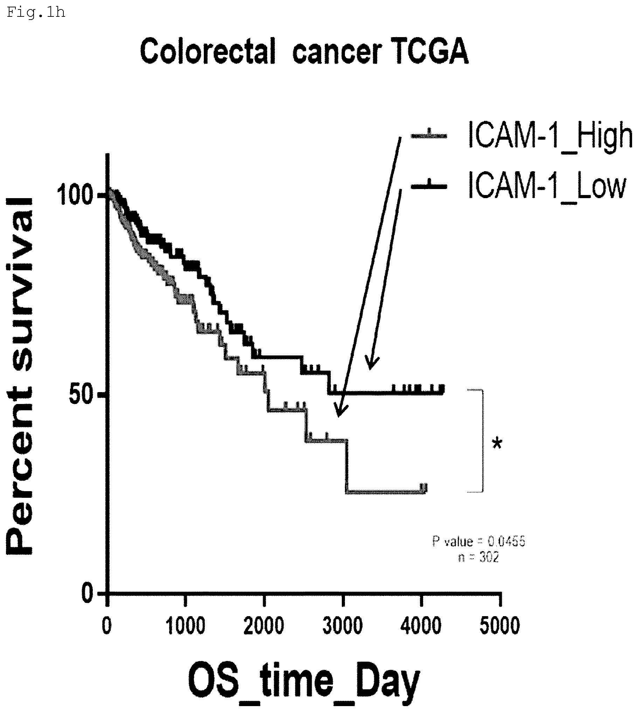

As a result of confirming the expression of ICAM-1 based on the GEO database, as shown in FIG. 1A, in GSE4183, the expression of ICAM-1 was high from the stage of inflammatory bowel disease (IBD), which is the stage of colorectal cancer formation. In GSE59071, it was confirmed that the expression of ICAM-1 was high in Crohn's disease and active ulcerative colitis.

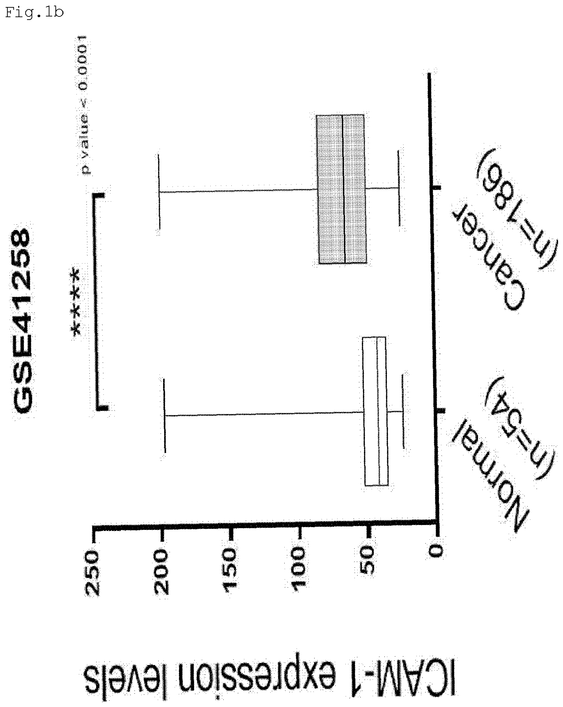

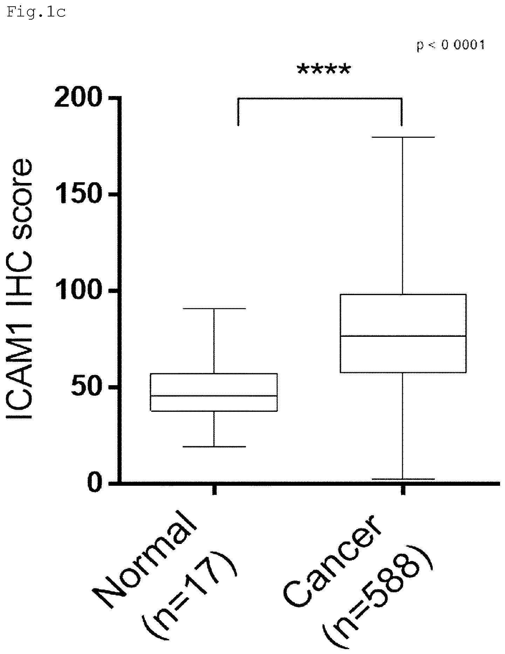

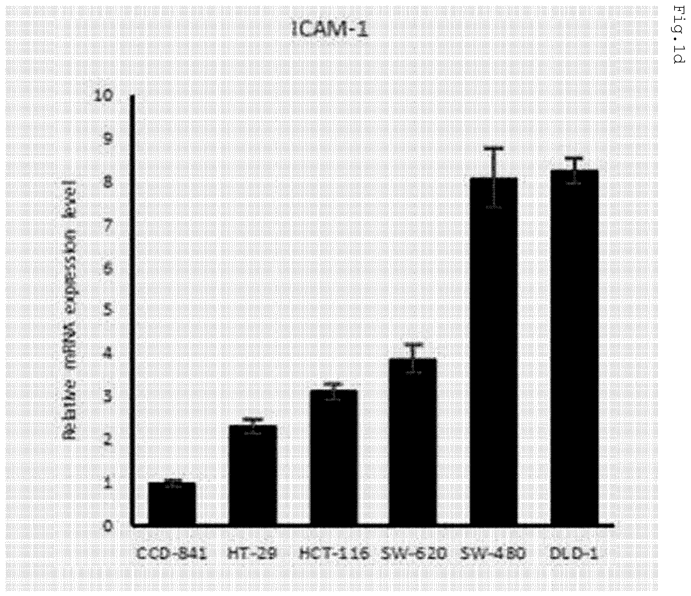

In addition, as a result of confirming the expression of ICAM-1 in GSE 41258, it was confirmed that the expression of ICAM-1 was higher in cancer patients than in normal subjects, as shown in FIG. 1B. When immunohistochemistry(IHC) was performed, as shown in FIG. 1C, it was confirmed that the expression of ICAM-1 was high in colorectal cancer patients. As a result of performing Real-time PCR on CCD841, a normal cell line, and colorectal cancer cell lines (HT-29, HCT-116, SW-620, SW-480, DLD-1) for confirming specific changes in the expression of ICAM-1, as shown in FIG. 1D, it was confirmed that the expression of ICAM-1 was increased in colorectal cancer cell lines.

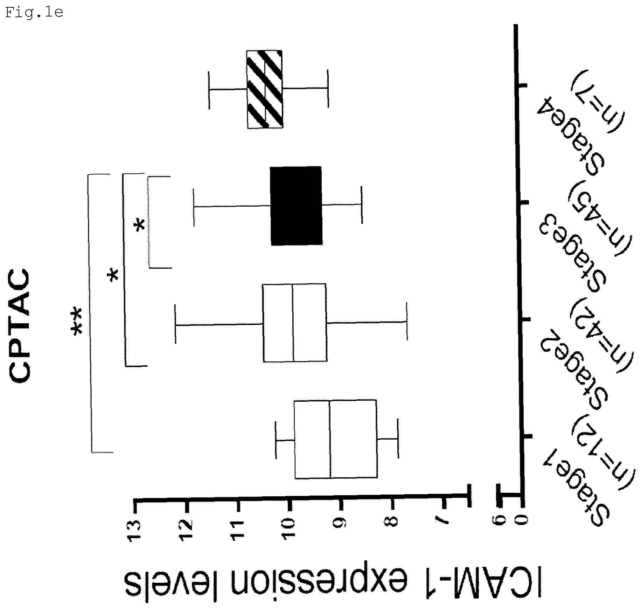

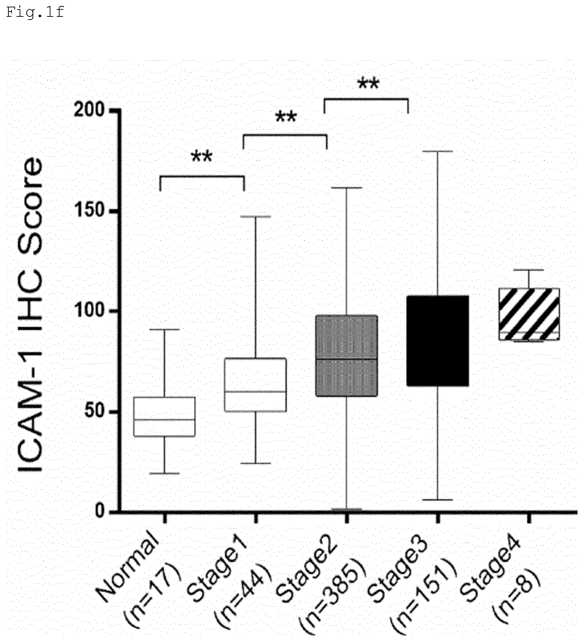

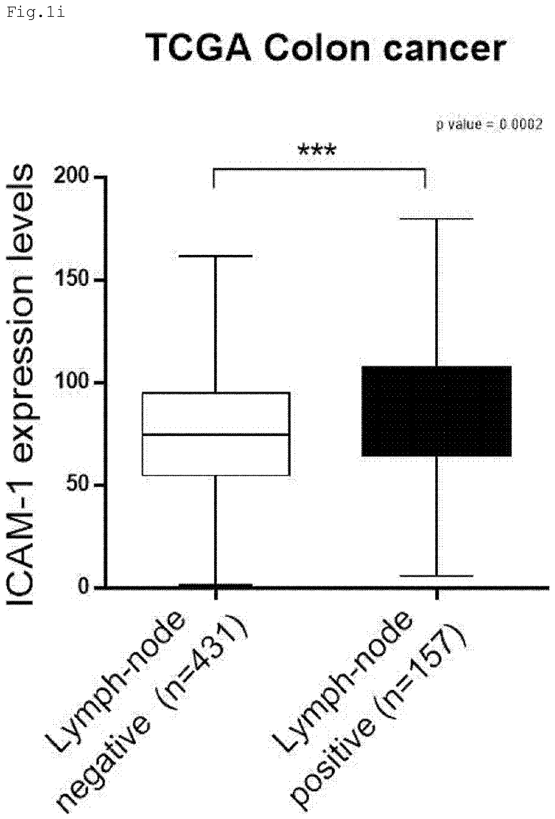

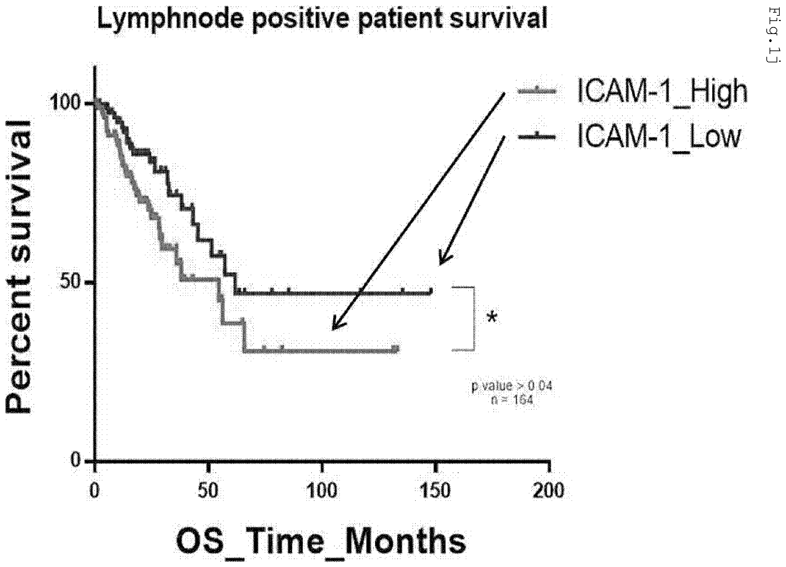

As shown in FIGS. 1E and 1F, it was confirmed in detail through the CPTAC database and the colorectal cancer patient tissue that the expression of ICAM-1 gradually increased as the malignant stage progressed. Through the TCGA database, disease-specific survival (DSS) and overall survival (OS) decrease as the expression level of ICAM-1 increases. As shown in FIG. 1I, it was specifically confirmed that the expression of ICAM-1 was further increased in patients with lymph node metastasis and that the prognosis was worse in patients with high ICAM-1 expression in patients with lymph node metastasis, as shown in FIG. 1J. Even in that case of patients with lymph node metastasis, the patients with high expression of ICAM-1 had a worse prognosis, as shown in FIG. 1J.

### Example 2. Confirmation of metastasis and angiogenesis of colorectal cancer cells according to ICAM-1 expression

In order to investigate the correlation between the expression of ICAM-1 and metastasis and angiogenesis, we confirmed that the change in invasibility and angiogenesis after suppressing or overexpressing ICAM-1 expression.

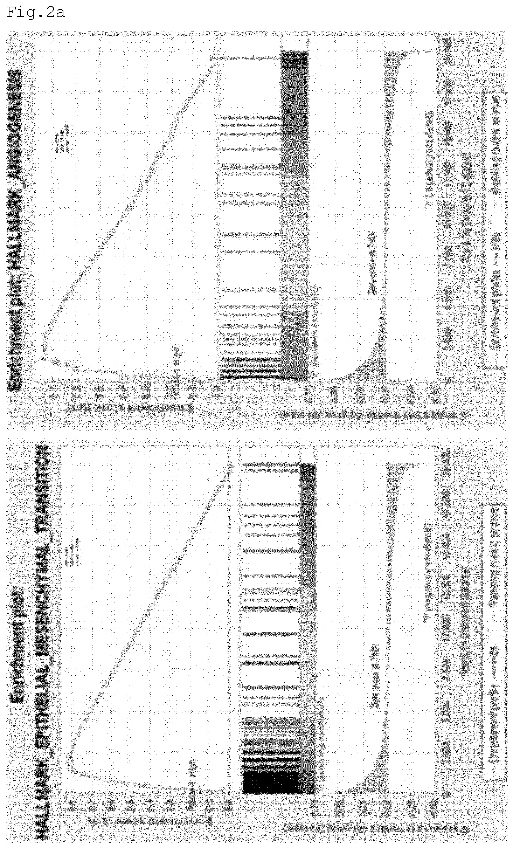

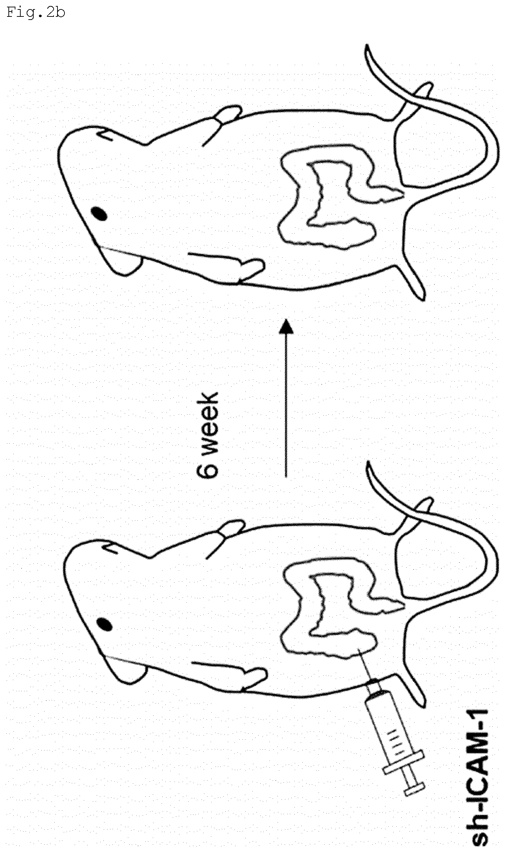

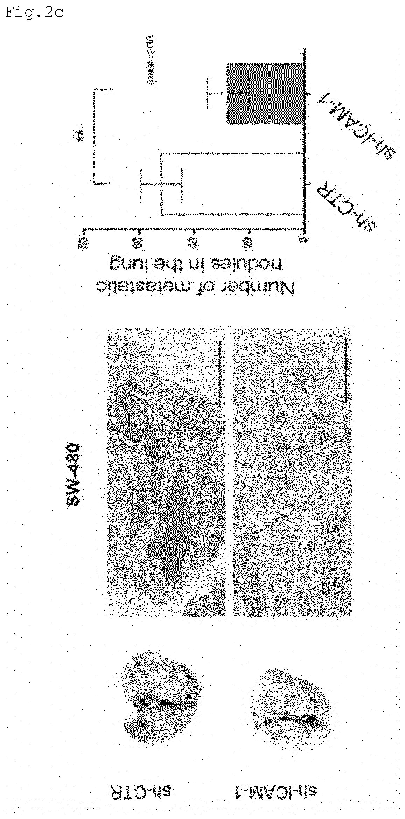

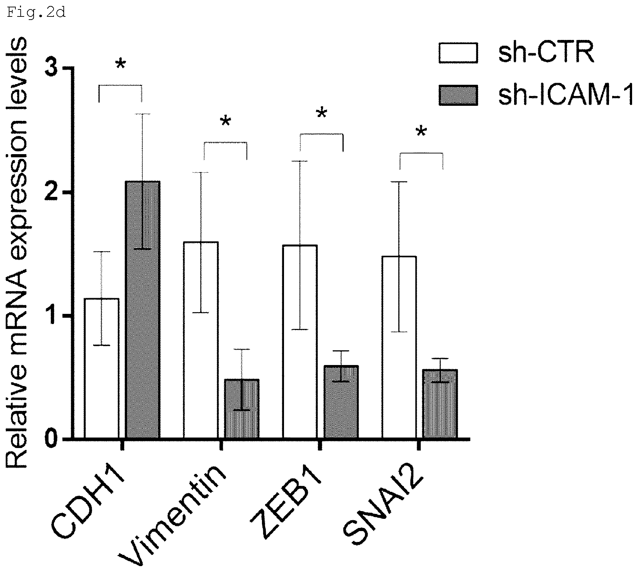

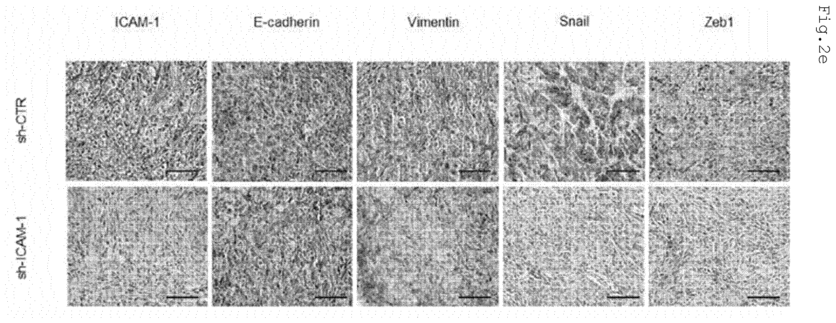

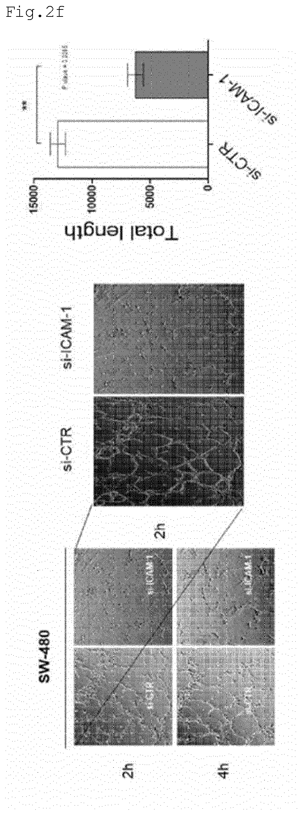

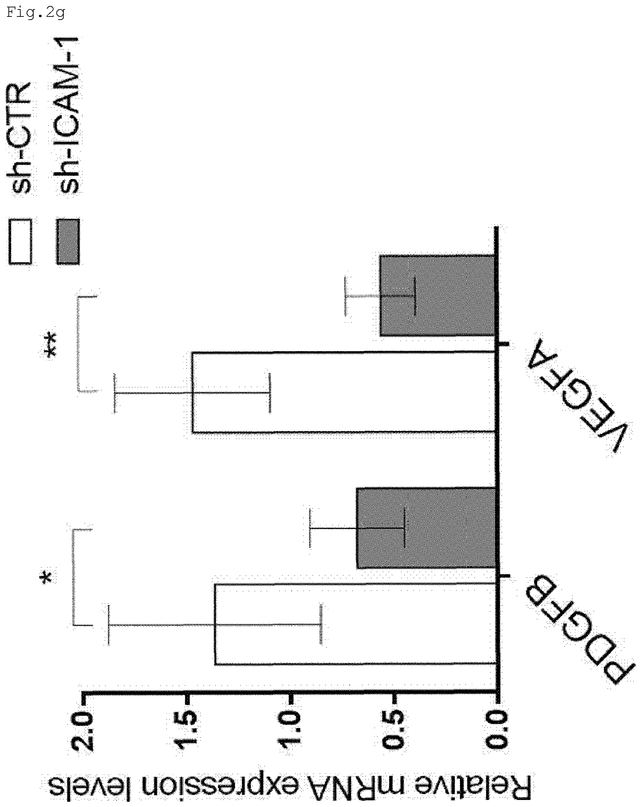

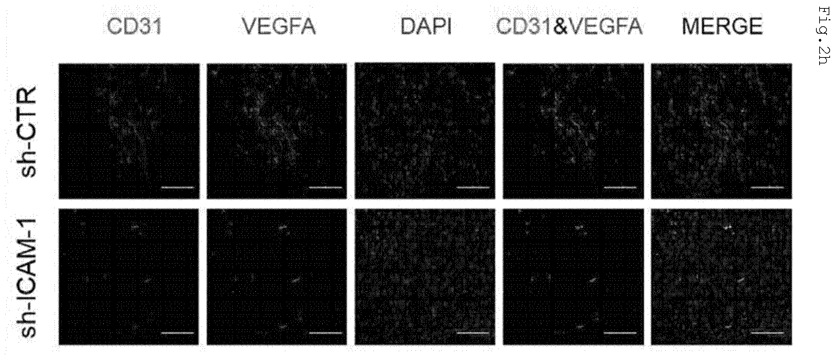

First, when confirming the relationship between tumor metastasis and angiogenesis in patients showing high expression of ICAM-1 through GSEA analysis, as shown in FIG. 2A, it was confirmed that the higher the expression of ICAM-1, the more tumor metastasis and angiogenesis increased. After preparing an SW-480 cell line with a low expression level of ICAM-1, as shown in FIG. 2B, it was injected into the cecum of a mouse, and tumor metastasis was observed. As shown in FIG. 2C, when the expression of ICAM-1 was low, the number of foci formed in the lungs was reduced, and when PCR and tissue immunostaining were performed, the expression level of metastasis related genes (E-cadherin, snail, and slug) was also reduced as shown in FIGS. 2D and 2E. When the expression level of factors promoting angiogenesis in vivo and the level of blood vessel generation were observed through PCR and tissue immunofluorescence staining, it was specifically confirmed that both angiogenesis and the expression of factors inducing angiogenesis were reduced when the expression level of ICAM-1 was reduced as shown in FIGS. 2G and 2H.

### Example 3. Confirmation of the mechanism of action of ICAM-1 and SRC

### 3-1. Confirmation of binding of ICAM-1 to SRC

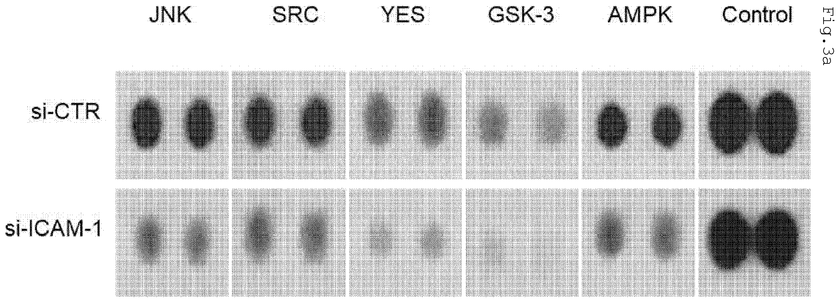

In order to confirm the signaling mechanism by which ICAM-1 regulates metastasis and angiogenesis in colorectal cancer cells, a phospho-kinase array was performed after ICAM-1 was inhibited. As shown in FIG. 3A, it was confirmed that the p-SRC decreased.

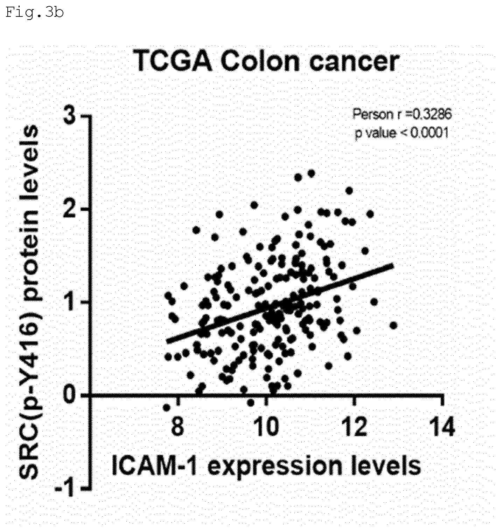

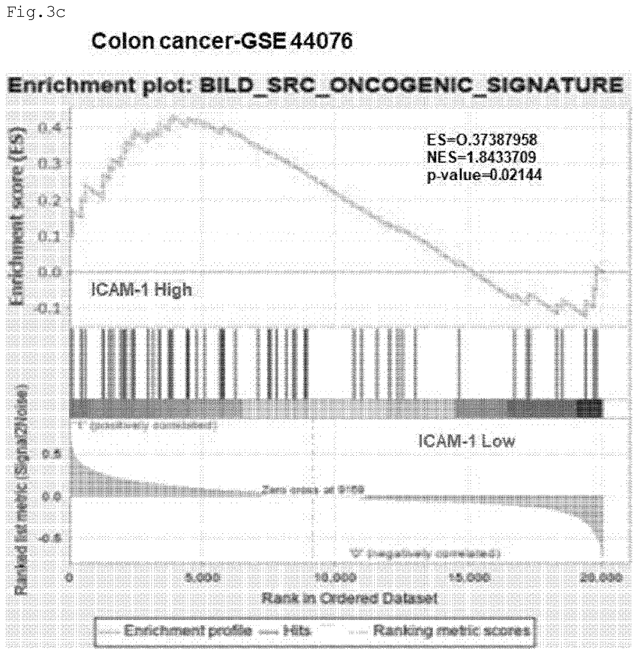

In addition, as a result of confirming the association between ICAM-1 expression and SRC activation through the TCGA database, the higher the expression of ICAM-1 is, the more the activity of SRC increases, as shown in FIG. 3B, and it was confirmed that the oncogene signature of SRC also increased as the expression level of ICAM-1 increased, as shown in FIG. 3C.

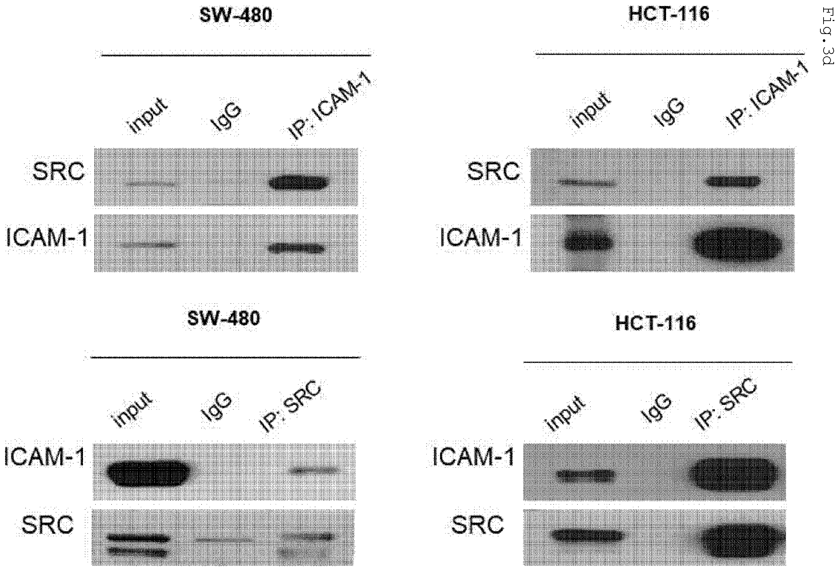

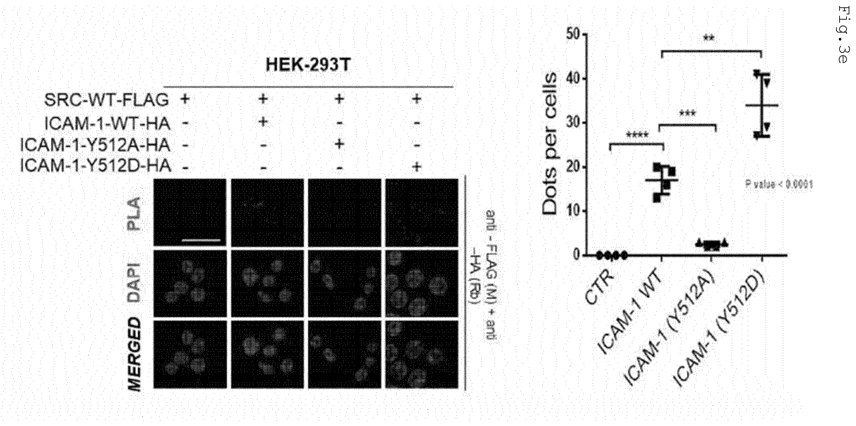

As a result of performing immunoprecipitation to confirm the expression correlation between ICAM-1 and SRC in SW-480 and HCT-116 colorectal cancer cells, it was confirmed that ICAM-1 binds to SRC endogenously, as shown in FIG. 3D. In particular, as shown in FIG. 3E, it was confirmed that SRC was bound to the Tyr residue site of ICAM-1.

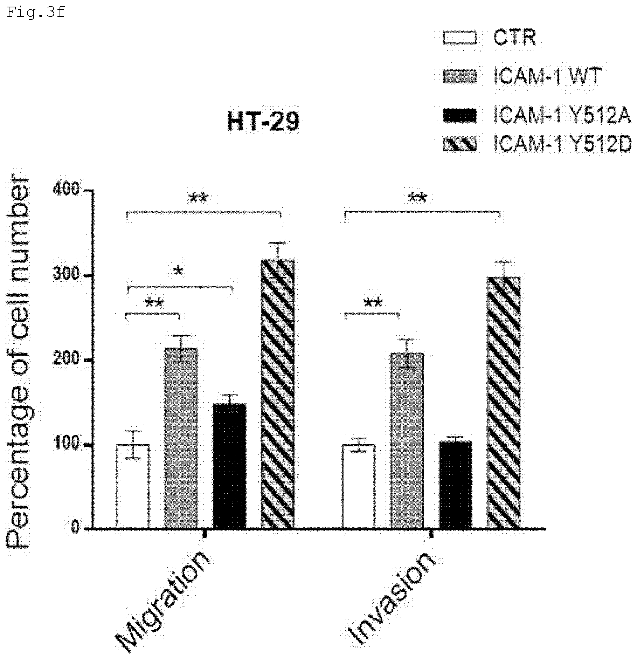

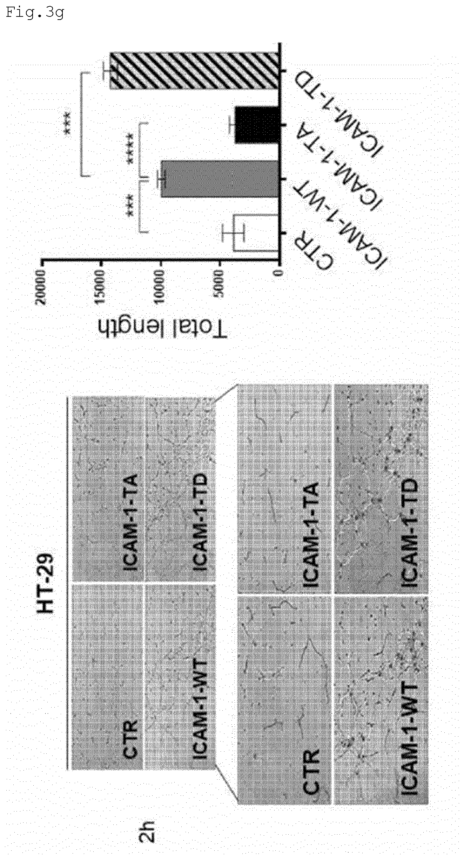

Furthermore, in order to confirm that ICAM-1 binds to SRC by a specific residue, an immunoprecipitation reaction was performed using a mutant with phosphomimetics form that inhibits (Y512A) or activates (Y512D) the Tyr512 phosphorylation residue of ICAM-1. As a result, as shown in FIGS. 3F and 3G, it was confirmed that the more activated the phosphoric residue, the stronger the binding thereto SRC.

### 3-2. Confirmation of the relevance of ICAM-1 and SRC with c-MET

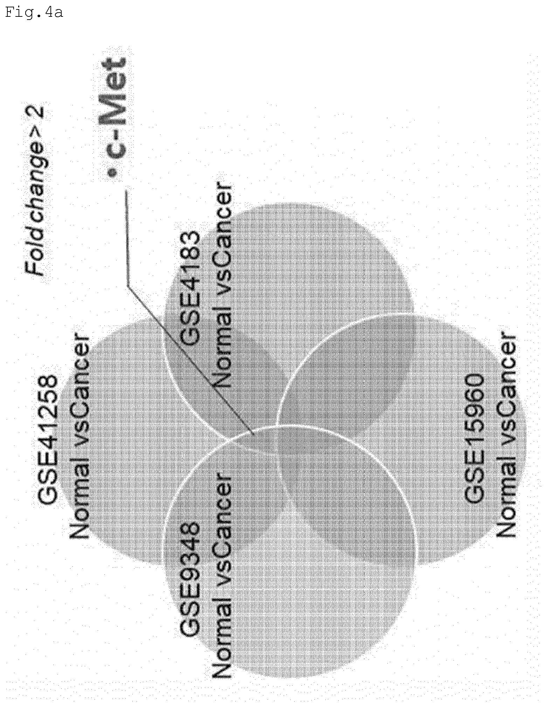

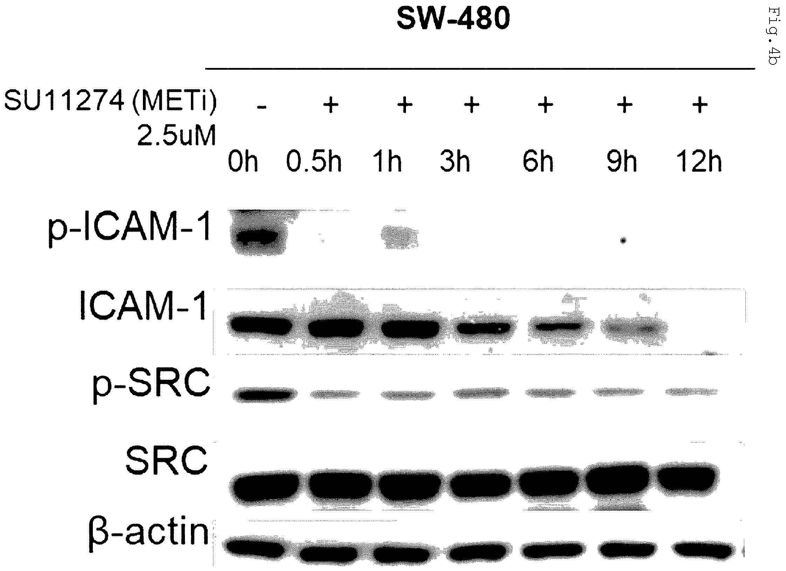

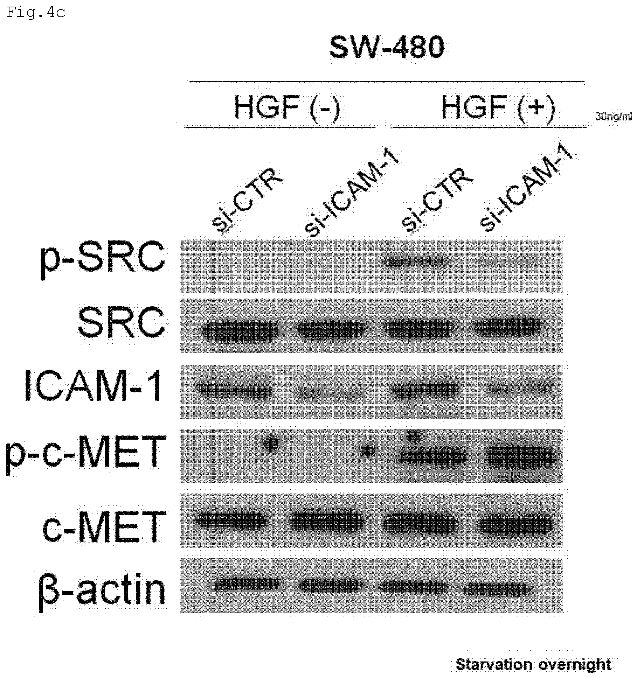

As a result of reviewing the four GEO databases, it was confirmed that the expression of c-MET was increased in all colorectal cancer patients, as shown in FIG. 4A, and based on these results, the role of c-MET in the mechanism of ICAM-1 and SRC was specifically confirmed. When the c-MET inhibitor was treated, as shown in FIG. 4B, it was confirmed that the phosphorylation of ICAM-1 and SRC is reduced. When HGF, the ligand of c-MET, was treated after the expression of ICAM-1 was decreased, the expression of p-SRC was decreased depending on the expression of ICAM-1, as shown in FIG. 4C.

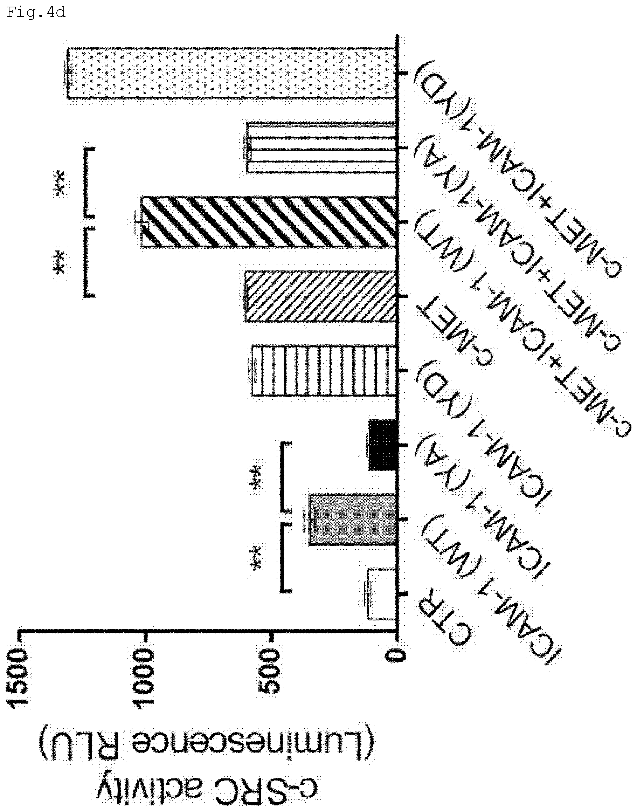

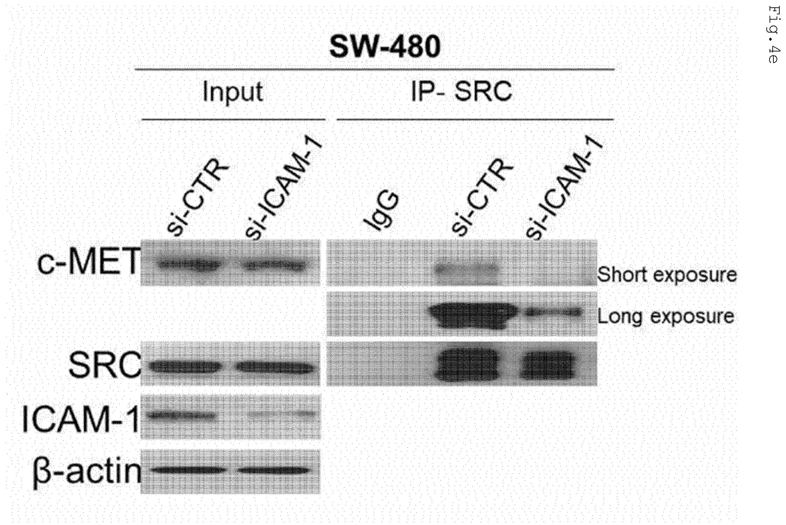

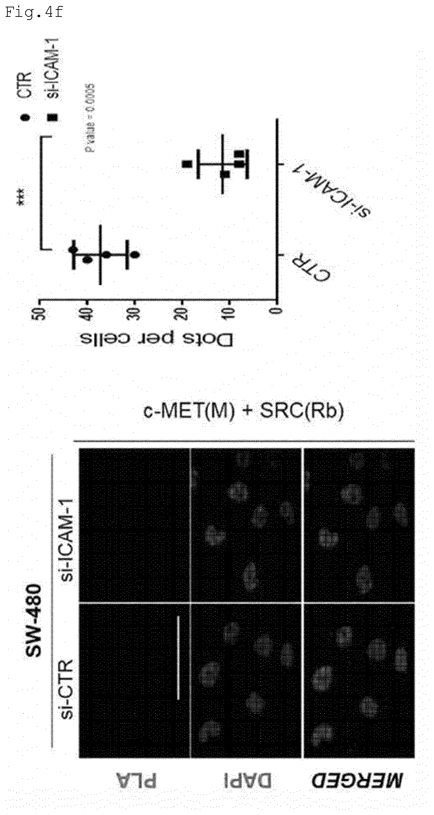

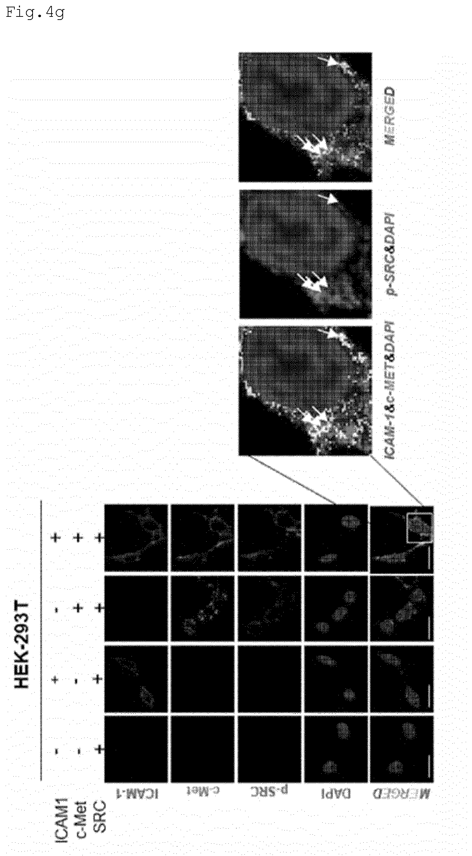

As a result of confirming the activation of SRC after constructing a mutant in which the ICAM-1 is inhibited or overexpressed in Example 3-1, as shown in FIG. 4D, it was confirmed that the activation of SRC in the mutant in which the ICAM-1 is overexpressed was increased to a significant level. In order to support the above results, as a result of performing immunoprecipitation and PLA staining, it was confirmed that, as shown in FIGS. 4E and 4F, as the expression of ICAM-1 decreases, the binding of c-MET to SRC is reduced. When ICAM-1 and c-MET were present at the same position, it was confirmed through ICC that p-SRC increased, and the results are shown in FIG. 4G.

### Example 4. Confirmation of metastatic ability and angiogenic ability of ICAM-1 dependent on SRC

It was confirmed whether ICAM-1 regulates cancer metastasis and angiogenesis dependent on SRC.

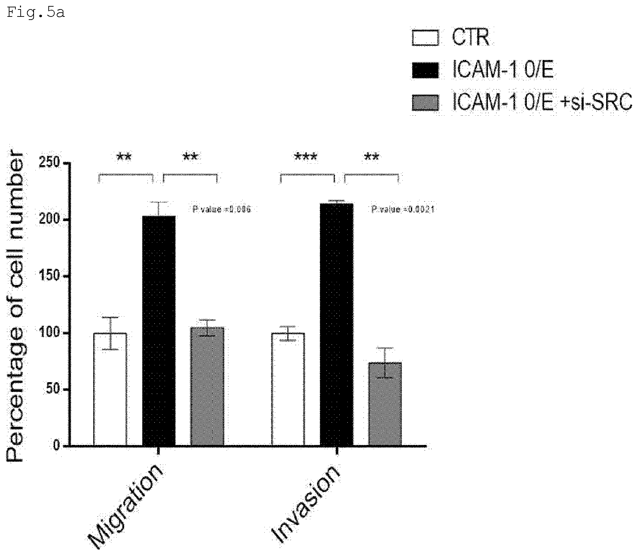

First, after overexpressing ICAM-1 and then knocking down SRC in a rescue experiment to confirm metastatic ability, as shown in FIG. 5A, even when ICAM-1 expression was increased, metastatic ability decreased as SRC decreased.

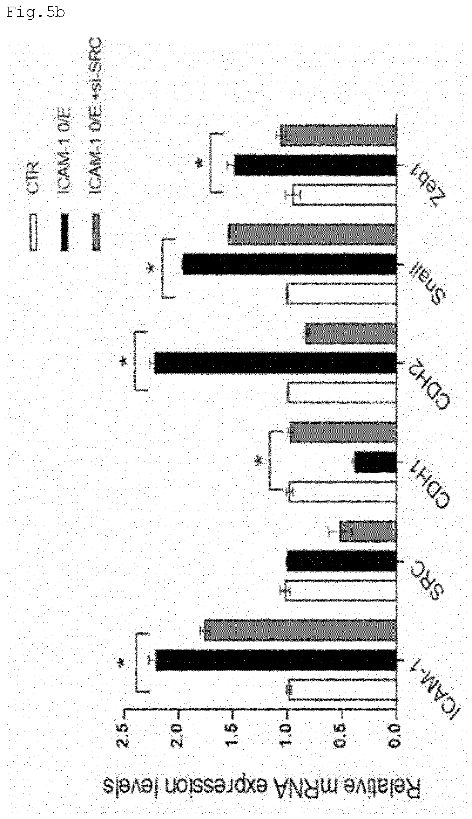

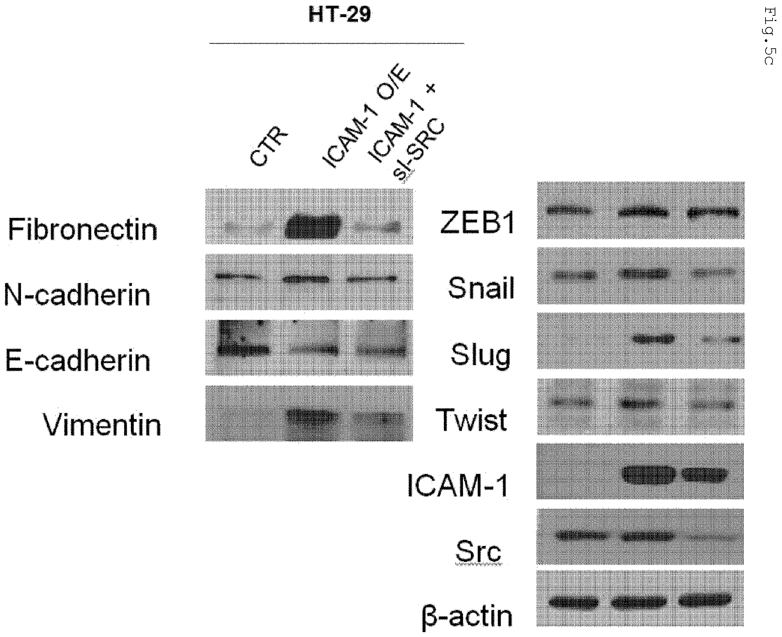

Next, as a result of confirming the expression of EMT markers and transcription factors using RT-PCR and western blot, it was confirmed that the expression of EMT markers and transcription factors decreased as the expression of SRC decreased, as shown in FIGS. 5B and 5C.

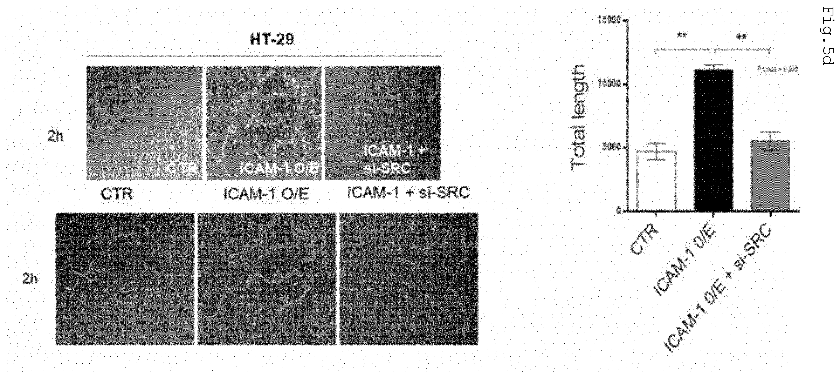

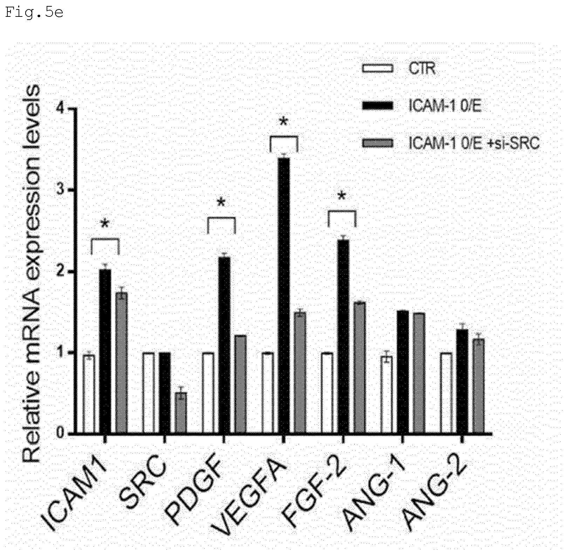

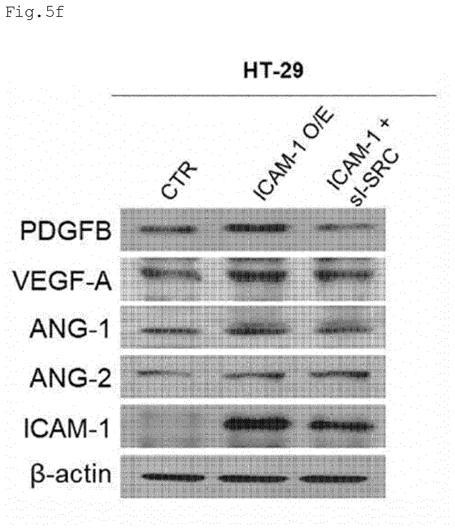

In addition, the blood vessels were photographed to confirm the expression of growth factors inducing angiogenesis in order to confirm the change in angiogenesis, and then, as a result of measuring the length, when ICAM-1 was overexpressed, and then SRC was knocked down, it was confirmed that angiogenesis was reduced (FIG. 5D), and the expression of factors involved in angiogenesis (FIG. 5E) and growth factors (FIG. 5F) were also decreased.

From the above results, it can be inferred that the metastatic ability and angiogenic ability of ICAM-1 are regulated depending on SRC.

### Example 5. Confirmation of patient survival rate and cancer malignancy according to the expression of ICAM-1

In addition to the in vitro experiment, to confirm whether the expression of ICAM-1 shows a correlation with colorectal cancer even when directly applied to a patient, the survival rate of colorectal cancer patients was checked through the TCGA database. As a result, as shown in FIG. 6A, it was confirmed that the survival rate of colorectal cancer patients was lowered when ICAM-1 was highly expressed.

In addition, as a result of checking the cancer grade of colorectal cancer patients according to ICAM-1 expression in GSE 17538, as shown in FIG. 6B, it was confirmed that there were more patients corresponding to stage 3 when ICAM-1 was higher than when ICAM-1 was lower.

Furthermore, the degree of cancer malignancy was confirmed through GDC TCGA, and at this time, it was indicated as a T stage, which is divided according to the tumor size among the TNM stages, and each stage is divided into T1, T2, T3, T4, and the higher the number, the more malignant cancer progresses. As a result, as shown in FIG. 6C, it was confirmed that the higher the expression of ICAM-1, the more patients corresponding to T3 and T4 appeared.

In order to specifically confirm the tumor malignant effect of ICAM-1 and SRC, that there is an interaction confirmed by Examples 3 and 4, the tissue of a colorectal cancer patient was observed. As shown in FIG. 6D, it was confirmed that ICAM-1 and p-SRC had a positive correlation, and when the prognosis was reviewed in detail, as shown in FIG. 6E, the higher the expression levels of ICAM-1 and p-SRC, the worse the prognosis was.

### Example 6. Confirmation of an effect of improvement in tumor malignancy by inhibition of ICAM-1 activity

When ICAM-1 inhibitor was administered, in order to confirm the effect of improving the metastasis of the tumor, the tumor was injected into the cecum and then treated with a monoclonal antibody targeting ICAM-1 when 3 weeks had elapsed, and the progress was confirmed. As a result, as shown in FIG. 7A, when the antibody was treated, it was confirmed that the metastasis of the tumor was reduced compared to the control group, and when PCR and tissue immunostaining were performed, as shown in FIGS. 7B and 7C, it was confirmed that the expression levels of CDH1, Vimentin, Snail, and Zeb1, which are genes involved in metastasis, were reduced.

When the SW-480 cell line was treated with a monoclonal antibody targeting ICAM-1, it was confirmed by measuring the imaging and length of blood vessels that the angiogenesis was significantly reduced (FIG. 7D). It was specifically confirmed that angiogenesis and the expression of growth factors controlling angiogenesis were reduced on in vivo, as shown in FIGS. 7E and FIG. 7F.

The above-described description of the present disclosure is for example, and it will be understood by those skilled in the art that the present disclosure may be easily modified in other specific forms without changing the technical spirit or essential features of the present disclosure. Therefore, it should be understood that the embodiments described above are exemplary and not limited in all respects.

### Industrial Applicability

The present disclosure showed that ICAM-1 can be a potential target for the development of colorectal cancer therapeutics by confirming that ICAM-1 can regulate cancer metastasis and angiogenesis in an SRC-dependent manner. Accordingly, when the target factor screened by the method of the present disclosure is used, it is expected to be useful in the industrial field for the development of a treatment for colorectal cancer.

## Claims

1. A method for screening colorectal cancer metastasis inhibitor, comprising the following steps:
treating a candidate material to cells in vitro;
measuring a binding level of ICAM-1 (Intracellular adhesion molecule 1) to SRC in the cell; and
selecting the candidate material which increases the binding level of ICAM-1 to SRC as a therapeutic material, compared to a non-treated group with the candidate material.

2. The method of claim 1,
wherein the cells are colorectal cancer.

3. The method of claim 1,
wherein the candidate material is selected from a group consisting of a compound, a microbial culture or extract, a natural extract, a nucleic acid, and a peptide.

4. The method of claim 3,
wherein the nucleic acid is selected from a group consisting of siRNA, shRNA, microRNA, antisense RNA, aptamer, LNA(locked nucleic acid), PNA(peptide nucleic acid), and morpholino.

5. The method of claim 1,
wherein the measuring the binding level of ICAM-1 to SRC comprises measuring a level of mRNA of ICAM-1 and SRC gene or a level of protein encoded by the gene.

6. The method of claim 5,
wherein the level of mRNA is measured through at least one method selected from the group consisting of a polymerase chain reaction(PCR), reverse transcription polymerase chain reaction(RT-PCR), real-time polymerase chain reaction(Real-time PCR), RNase protection assay(RPA), microarray, and northern blotting.

7. A composition for predicting a colorectal cancer metastasis, the composition comprising an agent for measuring a level of mRNA of ICAM-1 gene or an agent for measuring a level of a protein encoded by the gene.

8. A pharmaceutical composition for inhibiting colorectal cancer metastasis, the pharmaceutical composition comprising ICAM-1 gene or a protein encoded by the gene as an active ingredient.

9. A method for predicting a colorectal cancer metastasis, the method using a composition for predicting colorectal cancer metastasis according to claim 7.

10. A method of preventing, inhibiting or treating colorectal cancer metastasis, the method comprising administering a pharmaceutical composition of claim 8 to a subject.

11. Use of the composition for predicting colorectal cancer metastasis according to claim 7 for predicting colorectal cancer metastasis.

12. Use of the pharmaceutical composition of claim 8 for preventing, inhibiting or treating colorectal cancer metastasis.
